# EUROPEAN PATENT SPECIFICATION

(11) **EP 2 593 120 B1**
(45) Date of publication and mention of the grant of the patent: **08.03.2017**
(21) Application number: 11806135.7
(22) Date of filing: 14.07.2011
(51) Int. Cl.: A61K 38/08, A61P 31/18, A61K 38/22, A61K 38/07

(54) **THYMOSIN BETA PEPTIDES FOR USE IN THE TREATMENT OF HIV OR AIDS**
THYMOSIN BETA PEPTIDE ZUR BEHANDLUNG VON HIV ODER AIDS
PEPTIDES DE THYMOSINE BETA POUR LE TRAITEMENT DU SIDA OU DU VIH

(30) Priority: 14.07.2010 AU 2010903143
(43) Date of publication of application: 22.05.2013
(73) Proprietor: Adistem Ltd, Hong Kong (CN)
(72) Inventor: PASPALIARIS, Vasilis, Malvern East, Victoria 3145 (AU)
(74) Representative: Boult Wade Tennant
(86) International application number: PCT/AU2011/000878
(87) International publication number: WO 2012/006667

(56) References cited:
- EP-A1- 0 450 240
- EP-A1- 2 019 118
- WO-A1-03/035111
- WO-A2-03/020215
- WO-A2-2004/091550
- WO-A2-2006/076255
- WO-A2-2009/033816

## Description

The present disclosure relates to methods of treatment of HIV or AIDS.

### Background

Human immunodeficiency virus (HIV) is a lentivirus (a member of the retrovirus family) that causes acquired immunodeficiency syndrome (AIDS), a condition in humans in which the immune system begins to fail, leading to life-threatening opportunistic infections.

HIV infects primarily vital cells in the human immune system such as CD4⁺ T cells), macrophages and dendritic cells. HIV infection leads to low levels of CD4⁺ T cells through three main mechanisms: First, direct viral killing of infected cells; second, increased rates of apoptosis in infected cells; and third, killing of infected CD4⁺ T cells by CD8 cytotoxic lymphocytes that recognize infected cells. When CD4⁺ T cell numbers decline below a critical level, cell mediated immunity is lost, and the body becomes progressively more susceptible to opportunistic infections.

Most untreated people infected with HIV-1 eventually develop AIDS. These individuals mostly die from opportunistic infections or malignancies associated with the progressive failure of the immune system. HIV progresses to AIDS at a variable rate affected by viral, host, and environmental factors; most will progress to AIDS within 10 years of HIV infection: some will have progressed much sooner, and some will take much longer. Treatment with anti-retrovirals increases the life expectancy of people infected with HIV. Even after HIV has progressed to diagnosable AIDS, the average survival time with antiretroviral therapy was estimated to be more than 5 years as of 2005. Without antiretroviral therapy, someone who has AIDS typically dies within a year.

Current treatment for HIV infection consists of highly active antiretroviral therapy, or HAART. This has been highly beneficial to many HIV-infected individuals since its introduction in 1996, when the protease inhibitor-based HAART initially became available. Current HAART options are combinations (or "cocktails") consisting of at least three drugs belonging to at least two types, or "classes," of antiretroviral agents. Typically, these classes are two nucleoside analogue reverse transcriptase inhibitors (NARTIs or NRTIs) plus either a protease inhibitor or a non-nucleoside reverse transcriptase inhibitor (NNRTI).

Despite the success of HAART in controlling HIV infection and reducing HIV-associated mortality, current drug regimens are unable to completely eradicate HIV infection. Many people on HAART achieve suppression of HIV to levels below the limit of detection of standard clinical assays for many years. However, upon withdrawal of HAART, HIV viral loads rebound quickly with a concomitant decline in CD4+ T cells, which, in most cases, absent a resumption of treatment, leads to AIDS and death.

It is an aim of an embodiment of the present disclosure to provide a treatment for HIV or AIDs.

### Summary

A first aspect of the present disclosure provides a method of treating HIV or AIDS comprising administering a composition comprising a polypeptide comprising LKKTETQ (SEQ ID NO: 1) or a variant thereof.

An alternative first aspect provides a composition comprising a polypeptide comprising LKKTETQ (SEQ ID NO: 1) or a variant thereof for treating HIV or AIDS.

An alternative first aspect provides use of a composition comprising a polypeptide comprising LKKTETQ (SEQ ID NO: 1) or a variant thereof in the manufacture of a medicament for treating HIV or AIDS.

A second aspect provides a method of treating HIV or AIDS comprising administering a composition comprising a peptidomimetic of a polypeptide comprising amino acid sequence LKKTETQ (SEQ ID NO: 1) or a variant thereof.

An alternative second aspect provides a composition comprising a peptidomimetic of a polypeptide comprising amino acid sequence LKKTETQ (SEQ ID NO: 1) or a variant thereof for treating HIV or AIDs.

An alternative second aspect provides use of a composition comprising a peptidomimetic of a polypeptide comprising amino acid sequence LKKTETQ (SEQ ID NO: 1) or a variant thereof in the manufacture of a medicament for treating HIV or AIDs.

In one embodiment of the first aspect the polypeptide is LKKTETQ (SEQ ID NO:1) or Ac-LKKTETQ-OH or comprises a fragment of thymosin beta 4 comprising LKKTETQ (SEQ ID NO:1) or consists essentially of SEQ ID NO: 3.

In one embodiment of the second aspect the peptidomimetic is based on the polypeptide LKKTETQ (SEQ ID NO:1). In one embodiment of the second aspect the peptidomimetic is based on a thymosin beta 4 fragment thereof comprising LKKTETQ (SEQ ID NO:1) or a peptide consisting essentially of SEQ ID NO: 3.

### Detailed Description

The invention is defined by the claims. The present invention is based on the discovery that administration of the peptide of SEQ ID NO: 1, a peptide derived from thymosin beta 4 (Tβ4) , to HIV positive patients and those that had progressed to showing symptoms of AIDS increased CD4 and CD8 positive white blood cell counts and decreased viral load significantly.

The peptide of SEQ ID NO: 1 has no effect on T-cell tropic or macrophage tropic HIV isolates cultured in primary cells, indicating its anti-HIV activity is caused by an alternative mechanism.

Tβ4 is a small naturally occurring 43 amino acid peptide (SEQ ID NO: 2) first isolated from the thymus in 1981 and now identified in many tissues. Tβ4 has been implicated in many biological activities, including wound healing, angiogenesis, actin binding, cell migration and cell adhesion. It is anti-microbial, anti-apoptotic and has anti-inflammatory activity.

### MSDKPDMAEI EKFDKSKLKK TETQEKNPLP SKETIEQEKQ AGES (SEQ ID NO: 2)

Until the pesent disclosure it had not been disclosed or suggested that LKKTETQ (SEQ ID NO: 1) can be used for the treatment of HIV or AIDs.

In one embodiment the polypeptide does not comprise full length human wild type Tβ4 of SEQ ID NO: 2.

The polypeptide for use in the first and second aspects may include Tβ4, and/or Tβ4 isoforms, analogues or derivatives comprising SEQ ID NO: 1. or a variant thereof.

Variants or (SEQ ID NO: 1 are polypeptides in which at one or more amino acids positions there is an amino acid insertion, deletion or substitution, either conservative or non-conservative. There may be a mutation at one, two, three or four of the positions of the polypeptide sequence LKKTETQ (SEQ ID NO: 1).

A conservative substitution is a substitution of a similarly sized or charged residue for another, for example substitution of one residue in the following groups with another in the same group would be considered conservative:
Gly, Ala
Val, Ile, Leu
Asp, Glu
Asn, Gln,
Ser, Thr
Lys, Arg
Phe, Tyr

In one embodiment variants are based on the consensus sequence X₁LKX₂TX₃X₄X₅X₆ (SEQ ID NO: 3), wherein X is any amino acid. Preferably X is a conservative substitution of the native amino acid of Tβ4 given in SEQ ID NO: 2.

In one embodiment X₁ is 1, 2, 3, 4, or 5 amino acid residues or is absent, X₂ is K, H or A, X₃ is E or N, X₄ is T or M, X₅ is Q, N, E or A and X₆ is 1, 2, 3, 4 or 5 amino acid residues or is absent.
Preferably X₁ comprises (F/L)(D/N)(S/A/T/K/N)(K/N/G)
Preferably X₆ comprises (E/T)(K/E)(N/E).

The polypeptide may comprise one of amino acid sequences:
FDKSKLKKTETQEKN (SEQ ID NO: 4)
FDKAKLKKTETQEKN (SEQ ID NO: 5)
FDRSKLKKTETNTEE (SEQ ID NO: 6)
FDKTKLKKTETQEKN (SEQ ID NO: 7)
FDKSKLKKTNTEEKN (SEQ ID NO: 8)
FDRSKLKKTNTEEKN (SEQ ID NO: 9)
FDKTKLKKTETAEKN (SEQ ID NO: 10)
FNRAKLKKTETQEKN (SEQ ID NO: 11)
FNKAKLKKTEMQEKN (SEQ ID NO: 12)
FDAKKLKHTETNEKN (SEQ ID NO:13)
FNQNNLKHTETNEKN (SEQ ID NO:14)
LDKAKLKATEMQEKN (SEQ ID NO:15)
FDKAGLKKTETEEKE (SEQ ID NO:16).

In one embodiment the polypeptide consists essentially of the amino acid sequence provided in SEQ ID NO: 1 or in any one of SEQ ID NOs: 3-16.

In one embodiment the polypeptide has an N terminal acetyl group and a C terminal hydroxyl group, for example Ac-LKKTETQ-OH.

In another embodiment the polypeptide comprises or consists essentially of oxidized TB4, N-terminal variants of Tβ4, C-terminal variants of Tβ4, polypeptides or peptide fragments comprising or consisting essentially of the amino acid sequence LKKTETQ, LKKTETQ, LKKTNTQ, KLKKTETQ, LKKTETQQ, conservative variants thereof, or other peptide agents as described herein, having activity as described herein.

International Application Serial No. PCT/US99/17282, discloses LKKTET (SEQ ID NO: 17) and isoforms of Tβ4 which may be useful in accordance with the present disclosure as well as amino acid sequence LKKTETQ and conservative variants thereof, which may be utilized with the present disclosure.

International Application Serial No. PCT/GB99/00833 (WO 99/49883), discloses oxidized Tβ4 which may be utilized in accordance with the present disclosure.

Although the present disclosure is described primarily hereinafter with respect to SEQ ID NO: 1, it is to be understood that the following description is intended to be equally applicable to amino acid sequences provided as SEQ ID NOs: 3-17 and to Tβ4 and Tβ4 isoforms.

Many Tβ4 isoforms have been identified and have about 70%, or about 75%, or about 80% or more homology to the known amino acid sequence of Tβ4. Such isoforms include, for example, Tβ4^{ala}, Tβ9, Tβ10, Tβ11 , Tβ12, Tβ13, Tβ14 and Tβ15. Thus, it is specifically contemplated that known Tβ4 isoforms, such as Tβ4^{ala}, Tβ9, Tβ10, Tβ11 , Tβ12, Tβ13, Tβ14 and Tβ15, as well as Tβ4 isoforms not yet identified, will be useful in the methods of the present disclosure. The disclosure therefore further provides a composition comprising Tβ4 or fragments or variants or Tβ4 isoforms Tβ4^{ala}, Tβ9, Tβ10, Tβ11, Tβ12, Tβ13, Tβ14 and Tβ15 or fragments or variants for treating HIV or AIDS.

In one embodiment the composition is a pharmaceutical composition and comprises a pharamceutically acceptable carrier.

In another embodiment the composition is a veterinary composition and comprises a carrier suitable for veterinary use.

By the term "derivative thereof", we mean a peptide within which amino acids residues are replaced by residues (whether natural amino acids, non-natural amino acids or amino acid mimics) with similar side chains or peptide backbone properties.

Additionally, either one or both terminals of such peptides may be protected by N and C-terminal protecting groups, for example, groups with similar properties to acetyl or amide groups. It will be appreciated that the amino acid sequence may be varied, truncated or modified once the final peptide is formed.

Such derivatives may increase or decrease the peptide half-life in vivo. Examples of derivatives capable of increasing the half-life of peptide and polypeptides according to the invention include peptoid derivatives of the polypeptides, D-amino acid derivatives of the polypeptides, and peptide-peptoid hybrids.

The preparation of polypeptides is a routine process. For example, the polypeptide can be synthesised, and many companies offer the commercial synthesis of peptides.

Laboratory techniques are also well known for the preparation of peptides, such methods being readily performed by a skilled person; for example, using recombinant DNA technologies as set out in Sambrook et al (2001): Molecular cloning, a laboratory manual, 3rd edition, Cold Spring Harbor Press, Cold Spring Harbor, New York. Hence the skilled person can prepare thymosin β 4 polypeptides using the information provided herein and from common general knowledge.

The terms polypeptide and peptide herein are used interchangeably and no size restriction is intended when the term peptide is used instead of polypeptide or vice versa.

Peptides and polypeptides used according to the disclosure may be subject to degradation by a number of means (such as protease activity in biological systems). Such degradation may limit the bioavailability of the polypeptides and hence the ability of the polypeptides to achieve their biological function. There are wide ranges of well established techniques by which derivatives that have enhanced stability in biological contexts can be designed and produced. Such polypeptide derivatives may have improved bioavailability as a result of increased resistance to protease-mediated degradation.

In one embodiment, a derivative or analogue suitable for use according to the disclosure is more protease-resistant than the peptide from which it is derived.

The polypeptide may be made more protease-resistant by protecting the N and/or C terminal. For example, the N terminal may be protected by an acetyl group, or by an alkyl or aryl group, or an alkyl-CO- or aryl-CO- group, each of which may be optionally substituted. The C terminal may be protected by an amide group or by a substituted amide group.

Protease-resistance of a polypeptide derivative and the polypeptide from which it is derived may be evaluated by means of well-known protein degradation assays. The relative values of protease resistance for the polypeptide derivative and polypeptide may then be compared.

Peptoid derivatives of the polypeptides of the present disclosure may be readily designed from knowledge of the structure of the polypeptide. Commercially available software may be used to develop peptoid derivatives according to well-established protocols.

A further embodiment of a modified form of peptides according to the present disclosure comprises D-amino acid forms of the peptide. The preparation of peptides using D-amino acids rather than L-amino acids greatly decreases any unwanted breakdown of such an agent by normal metabolic processes, decreasing the amounts of agent which need to be administered, along with the frequency of its administration.

Peptides, analogues, or derivatives represent products that may advantageously be expressed by biological cells and the disclosure includes use of such agents produced recombinantly.

The term "peptidomimetic" refers to a compound that mimics the conformation and desirable features of a particular peptide as a therapeutic agent, but that avoids the undesirable features. For example, morphine is a compound which can be orally administered, and which is a peptidomimetic of the peptide endorphin. There are a number of different approaches to the design and synthesis of peptidomimetics, as is well known in the art.

The peptides can also contain further amino acid sequences which are not derived from the amino acid sequence of thymosin: for example, other amino acid sequences which provide a separate function of the peptide (such as a tag, or a catalytic domain). Such peptides are also included in the aspects of the disclosure.

All peptides, polypeptides, fragments, variants, derivatives or peptidomimetics of Tβ4 comprising or based on SEQ ID NO:1 or a variant thereof are hereinafter referred to as therapeutic agent(s). Such therapeutic agents are all capable of reducing CD4 positive cell levels in subjects infected with HIV or AIDS.

As can be appreciated, the medicament may be administered to a variety of different subjects. By "subject" we include any animal that is susceptible to developing HIV or AIDS or who is infected with the disease, preferably a vertebrate, more preferably a mammal such as a domesticated or farmyard animal or a human. Most preferably the subject is a human.

The therapeutic agent may be administered orally, topically, or parenterally in medicaments containing conventional non-toxic pharmaceutically acceptable carriers, adjuvants, and vehicles.

The term parenteral as used herein includes intravenous, intraarterial, intraperitoneal, intramuscular, subcutaneous, subconjunctival, intracavity, transdermal and subcutaneous injection, aerosol for administration to lungs or nasal cavity or administration by infusion by, for example, osmotic pump.

Various means by which a medicament comprising a therapeutic agent can be formulated are provided below.

The therapeutic agent may be formulated into compositions having a number of different forms depending, in particular on the manner in which the composition is to be used. Thus, for example, the composition may be in the form of a powder, tablet, capsule, liquid, ointment, cream, gel, hydrogel, aerosol, spray, micelle, transdermal patch, liposome or any other suitable form that may be administered to a person or animal. It will be appreciated that the vehicle for the polypeptide should be one which is well tolerated by the subject to whom it is given, and preferably enables delivery of the therapeutic agents to the target cell, tissue, or organ.

Hence, it is preferred that that polypeptide is delivered by means of a suitably protected carrier particle, for example, a micelle.

The therapeutic agents may be used in a number of ways. For instance, systemic administration may be required in which case therapeutic agents may be contained within a composition which may, for example, be ingested orally in the form of a tablet, capsule or liquid. It is preferred that therapeutic agents are administered by injection into the blood stream. Injections may be intravenous (bolus or infusion) or subcutaneous (bolus or infusion).

Therapeutic agents may be combined in pharmaceutical compositions having a number of different forms depending, in particular on the manner in which the composition is to be used. Thus, for example, the composition may be in the form of a powder, tablet, capsule, liquid, ointment, cream, gel, hydrogel, aerosol, spray, micelle, transdermal patch, liposome or any other suitable form that may be administered to a person or animal. It will be appreciated that the vehicle used to provide the treatment should be one which is well tolerated by the subject to whom it is given, and preferably enables delivery of the therapeutic to the target cell, tissue, or organ.

In a preferred embodiment, the pharmaceutical vehicle is a liquid and the pharmaceutical composition is in the form of a solution. In another embodiment, the pharmaceutical vehicle is a gel and the composition is in the form of a cream or the like.

Therapeutic agents may also be incorporated within a slow or delayed release device. Such devices may, for example, be inserted on or under the skin, and the therapeutic agent may be released over weeks or even months. Such devices may be particularly advantageous when long term treatment with the therapeutic agents is required and which would normally require frequent administration (e.g. at least daily injection).

It will be appreciated that the amount of a therapeutic agent that is required is determined by its biological activity and bioavailability which in turn depends on the mode of administration, the physicochemical properties of the therapeutic agent employed, and whether the therapeutic agent is being used as a mono-therapy or in a combined therapy. Also, the amount will be determined by the number and state of target cells to be treated. The frequency of administration will also be influenced by the above-mentioned factors and particularly the half-life of the therapeutic agent within the subject being treated.

Optimal dosages to be administered may be determined by those skilled in the art, and will vary with the particular therapeutic agent in use, the strength of the preparation, the mode of administration, and the advancement of the disease condition. Additional factors depending on the particular subject being treated will result in a need to adjust dosages, including subject age, weight, gender, diet, and time of administration.

Known procedures, such as those conventionally employed by the pharmaceutical industry (e.g. in vivo experimentation, clinical trials, etc.), may be used to establish specific formulations of therapeutic agents and precise therapeutic regimes (such as daily doses of the therapeutic agents and the frequency of administration).

Daily doses may be given as a single administration (e.g. a single daily injection).

Alternatively, the therapeutic agents used may require administration twice or more times during a day. As an example, an therapeutic agents may be administered as two (or more depending upon the severity of the condition) daily doses of between 1 mg and 1000 mg (i.e. assuming a body weight of 70kg). A patient receiving treatment may take a first dose upon waking and then a second dose in the evening (if on a two dose regime) or at 3 or 4 hourly intervals thereafter. Alternatively, a slow release device may be used to provide optimal doses to a patient without the need to administer repeated doses.

In one embodiment the medicament comprising the therapeutic agent is administered subcutaneously or is formulated for subcutaneous administration to a subject. In one embodiment the medicament is an injectable composition.

In one embodiment the medicament is formulated to provide between 1 to 1000 mg of the therapeutic agent per administration.

A fifth aspect provides a population of stem cells that secrete thymosin β4 for use as a medicament for the prevention or treatment HIV or AIDS. Until the present disclosure, it had not been disclosed or suggested that such stem cells would have this utility.

Stem cells are cells that have the potential to differentiate into a number of cell types in the body. Theoretically, stem cells may divide without limit to replenish other cells for as long as the organism is alive. Upon differentiation, the daughter cell has the potential to remain a stem cell or become another cell type, for example lung cell and display its characteristics, thus holding promise for many 5 diseases by replacing damaged tissues. These phenomena may be induced under specific physiological and experimental conditions.

In general, stem cell therapy represents a therapeutic method by which degenerative and/progressive diseases (such as those caused by premature death or malfunction of cell types that the body is unable to replace) may be treated. It is hoped that addition of stem cells may help nucleate and promote the development of functional cells and/or tissues to replace those lost, thereby restoring normal healthy activity/function.

For the purposes of the present disclosure "stem cells" are taken to comprise nullipotent, totipotent or pluripotent cells, and progenitor cells (or precursor cells) to comprise multipotent cells. For the avoidance of doubt, the medicament and methods of the disclosure can comprise a therapeutically effective quantity of either stem or progenitor cells, or both stem and progenitor cells.

A suitable source of stem cells that may be used are cells derived from the inner cell mass/epiblast of pre-implantation embryos. Such embryonic stem (ES) cells are readily obtainable and are capable of giving rise to all possible embryonic and adult cell lineages. In particular, the undifferentiated human ESC (H1 line from WiCell Research Institute, Inc, Madison, WI: www.wicell.org) could be used in the disclosure this cell line is commercially available.

A further source of stem cells that can be used are umbilical cord-derived cells. A still further source of stem cells, are those isolated from adult tissues, including adipose tissues.

Where the medicament involves the use of biological cells, preferably the formulation for comprises biological cells in a suitable liquid carrier. Such a liquid carrier is preferably non-immunogenic, and may comprise a saline solution, cell culture medium, or distilled water. Formulations for injection may be as described above, or may also be provided in the form of a gel, which may preferably be capable of resolution by the body of the subject treated. Formulations suitable for implantation may take the forms described for injection or inhalation, and may also comprise biological cells provided in a scaffold or matrix capable of providing a foundation for new tissue development.

The methods of the disclosure are especially useful for the treatment of AIDS and related clinical conditions such as AIDS related complex (ARC), progressive generalized lymphadenopathy (PGL), Kaposi's sarcoma, thromobocytopenic purpura, AIDS-related neurological conditions such as AIDS dementia complex, multiple sclerosis or tropical paraperesis, anti-HIV antibody-positive and HIV-positive conditions, including such conditions in asymptomatic patients.

As well as treating AIDS or HIV the present disclosure can also be used to treat or prevent the symptoms or effects of a viral infection in an infected patient. In one embodiment the viral infection is a retroviral infection, in particular an HIV infection.

The treatment in accordance with the present disclosure may be supplemented with treatment with another therapeutic agent. The treatments may be administered simultaneously (i.e., concurrently) in either the same or different pharmaceutical compositions or sequentially in any order. The amounts of the active ingredient(s) and pharmaceutically active agent(s) and the relative timings of administration will be selected in order to achieve the desired combined therapeutic effect.

Examples of other therapeutic agents include nucleotide reverse transcriptase inhibitors such as zidovudine, didanosine, lamivudine, zalcitabine, abacavir, stavidine, adefovir, adefovir dipivoxil, fozivudine, todoxil, emtricitabine, alovudine, amdoxovir, elvucitabine, and similar agents; non-nucleotide reverse transcriptase inhibitors (including an agent having anti- oxidation activity such as immunocal, oltipraz, etc.) such as nevirapine, delavirdine, efavirenz, loviride, immunocal, oltipraz, capravirine, TMC-278, TMC-125, etravirine, and similar agents; protease inhibitors such as saquinavir, ritonavir, indinavir, nelfinavir, amprenavir, fosamprenavir, brecanavir, raltegravir, atazanavir, tipranavir, palinavir, lasinavir, and similar agents; entry inhibitors such as enfuvirtide (T-20), T-1249, PRO-542, PRO-140, TNX-355, BMS-806, 5-Helix and similar agents; lntegrase inhibitors such as L-870,810, raltegravir and similar agents; Budding inhibitors such as PA-344 and PA-457, and similar agents; and CXCR4 and/or CCR5 inhibitors such as vicriviroc (Sch-C), Sch-D, TAK779, maraviroc (UK 427,857), TAK449 and similar agents.

In one embodiment the method comprises administering the therapeutic agent a subject being treated with another therapeutic agent as listed above or otherwise.

In one embodiment the therapeutic agent is for administering to a subject being treated with another therapeutic agent as listed above or otherwise.

In an embodiment the therapeutic agent and other therapeutic agent act synergistically in the treatment of HIV or AIDS.

The terms "treating" and "treatment" as used herein refer to reduction in severity and/or frequency of symptoms, elimination of symptoms and/or underlying cause, prevention of the occurrence of symptoms (prophylaxis) and/or their underlying cause, and improvement or remediation of damage. Thus, for example, the present method of "treating" a disorder encompasses both prevention of the disorder in a predisposed individual and treatment of the disorder in a clinically symptomatic individual.

"Treating" as used herein covers any treatment of, or prevention of a condition in a vertebrate, a mammal, particularly a human, and includes: inhibiting the condition, i.e., arresting its development; or relieving or ameliorating the effects of the condition, i.e., cause regression of the effects of the condition.

"Prophylaxis" or "prophylactic" or "preventative" therapy or "prevent" or "prevention" as used herein includes preventing the condition from occurring or ameliorating the subsequent progression of the condition in a subject that may be predisposed to the condition, but has not yet been diagnosed as having it.

In one embodiment the method or composition prevents or slows progression from HIV infection to AIDS. In another embodiment the method or composition lessens the symptoms of AIDS. In another embodiment the method or composition improve immune response in subjects with AIDS. In another embodiment the method or composition improve quality of life for subjects with AIDS. In another embodiment the method or composition prolong life span in subjects with AIDS.

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

It must also be noted that, as used in the subject specification, the singular forms "a", "an" and "the" include plural aspects unless the context clearly dictates otherwise.

The invention is now further described in detail by reference to the following example. The example is provided for purposes of illustration only, and is not intended to be limiting unless otherwise specified.

### Example 1:

LKKTETQ (SEQ ID NO:1) is a fragment of Thymosin B4 (Tβ4).

The peptide Ac-LKKTETQ-OH was the active therapeutic agent investigated in a pilot trial on twenty similar HIV positive men and women, some who had progressed to severe AIDS.

Each man or women was injected subcutaneously with a composition comprising the peptide Ac-LKKTETQ-OH (10mg), once a week for 12 weeks. Blood tests for CD4 and CD8-positive cells and HIV viral load (by PCR) and routine pathology were conducted at baseline, 8 weeks and 12 weeks. The results are displayed in the three Tables below. Hemoglobin levels, Creatinine, ALT, AST, Alkaline Phos, Bilirubin, GGT, Albumin, Total Protein and globulin tests did not show anything remarkable. A small drop in blood glucose was seen but this was not determined to be significant. No adverse effect has been reported so far.

**Table 1. WEIGHT**

| Patient | Sex | Weight | | |
|---|---|---|---|---|
| | | Initial | 8 weeks | 12 weeks |
| A | M | 44 | 55 | 58 |
| B | F | 67 | 70 | 70.5 |
| C | M | 74 | 78 | 78.5 |
| D | F | 75 | 78 | 78.5 |
| E | F | 88 | 92 | 92 |
| F | M | 47 | 52 | 52 |
| G | M | 52 | 59 | 72 |
| H | M | 66 | 71 | 72 |
| I | F | 78 | 82 | 82 |
| J | F | 76 | 79 | 79 |
| K | F | 67 | 72 | 71 |
| L | F | 60 | 60.5 | 71 |
| M | F | 48 | 50.5 | 51 |
| N | M | 48 | 58 | 65 |
| O | F | 71 | 72 | 72 |
| P | F | 78 | 79 | 80 |
| Q | F | 69 | 71.5 | 72 |
| R | M | 67 | 68.5 | 69 |
| S | F | 58 | 61 | 60 |
| T | F | 55 | 56 | 55 |

**Table 2: CD4-positive and CD8-positive white blood cell counts**

| Patient | CD4 | | |
|---|---|---|---|
| | Initial | 8 weeks | 12 weeks |
| A | 110 | 300 | 430 |
| B | 262 | 316 | 416 |
| C | 496 | 602 | 675 |
| D | 732 | 816 | 879 |
| E | 146 | 415 | 604 |
| F | 96 | 158 | 175 |
| G | 130 | 407 | 425 |
| H | 292 | 456 | 625 |
| I | 383 | 477 | 662 |
| J | 126 | 348 | 404 |
| K | 127 | 381 | 498 |
| L | 169 | 439 | 510 |
| M | 317 | 301 | 424 |
| N | 119 | 335 | 452 |
| O | 595 | 543 | 749 |
| P | 595 | 428 | 616 |
| Q | 269 | 466 | 602 |
| R | 249 | 259 | 368 |
| S | 200 | 471 | 469 |
| T | 196 | 466 | 571 |

| Patient | CD8 | | |
|---|---|---|---|
| | Initial | 8 weeks | 12 weeks |
| A | 101 | 980 | 1330 |
| B | 318 | 360 | 459 |
| C | 1059 | 1310 | 1718 |
| D | 688 | 1379 | 1394 |
| E | 321 | 1044 | 1339 |
| F | 1310 | 1296 | 1226 |
| G | 208 | 959 | 1190 |
| H | 1031 | 2120 | 2647 |
| I | 759 | 866 | 958 |
| J | 652 | 773 | 800 |
| K | 278 | 594 | 712 |
| L | 621 | | 893 |
| M | 786 | 606 | 983 |
| N | 245 | 939 | 1034 |
| O | 613 | 617 | 880 |
| P | 612 | 648 | 890 |
| Q | 247 | 456 | 521 |
| R | 618 | 1108 | 1384 |
| S | 323 | 1125 | 1201 |
| T | 407 | 594 | 660 |

**Table 3: HIV Viral Load**

| Patient | HIV Viral Load (copies/ml) | | |
|---|---|---|---|
| | Initial | 8 weeks | 12 weeks |
| A | 214000 | 200 | undetectable |
| B | 50809 | 98687 | 5151 |
| C | 120 | 24 | undetectable |
| D | 10130 | undetectable | undetectable |
| E | 223767 | undetectable | undetectable |
| F | 256228 | 28410 | undetectable |
| G | 257000 | 450 | undetectable |
| H | 114594 | 8214 | undetectable |
| I | 59509 | 17616 | undetectable |
| J | 38988 | undetectable | undetectable |
| K | 128685 | 15201 | undetectable |
| L | 53814 | undetectable | undetectable |
| M | 139786 | undetectable | undetectable |
| N | 144000 | 145 | undetectable |
| O | 13803 | undetectable | undetectable |
| P | 5060 | undetectable | undetectable |
| Q | 3567 | undetectable | undetectable |
| R | 52915 | undetectable | undetectable |
| S | 149212 | 12004 | undetectable |
| T | 115281 | 8781 | undetectable |

### Example 2:

The peptide Ac-LKKTETQ-OH was evaluated in fresh human peripheral blood mononuclear cells (PBMCs) against CXCR4 coreceptor-trophic HT/92/599 and the CCR5 coreceptor-trophic BaL subtype B strains of HIV-1. The peptide was added to the cells at 8 hours prior to infection and immediately prior to infection. The AZT control compound was evaluated in parallel with the peptide and yielded EC₅₀ values of 8 and 6 nM against HIV-1_{HT/92/599} and values of 6 and 10 nM against HIV-1_{BaL}. The peptide of SEQ ID NO:1 did not demonstrate antiviral activity in the PBMC assays when evaluated at concentrations up to a high test concentration of 500 µM.

The results of the pilot trial were very encouraging. The key parameters of HIV viral load, CD4 positive and CD8 positive white cell blood counts, and weight gain have all shown extremely significant improvements from the baseline - perhaps the most significant is the HIV viral load which, in one patient, has gone from levels of 257000 to undetectable over the 12 week period of the pilot trial.

## Claims

1. A composition comprising a polypeptide for use in the treatment of HIV or AIDS, wherein the polypeptide is LKKTETQ (SEQ ID NO: 1), Ac-LKKTETQ-OH or X₁LKX₂TX₃X₄X₅X₆ (SEQ ID NO: 3)and wherein X is any amino acid.

2. The composition for use according to claim 1, wherein the composition is administered to a subject being treated with another therapeutic agent.

3. The composition for use according to claim 2, wherein the other therapeutic agent is a nucleotide reverse transciptase inhibitor, a non-nucleotide reverse transcriptase inhibitor, a protease inhibitor, an entry inhibitor, an integrase inhibitor, or a budding inhibitor.

4. The composition for use according to claim 1, wherein the subject is being treated with a plurality of other therapeutic agents.

## Patentansprüche

1. Zusammensetzung umfassend ein Polypeptid zur Verwendung in der Behandlung von HIV oder AIDS, wobei das Polypeptid LKKTETQ (SEQ ID NO: 1), Ac-LKKTETQ-OH oder X₁LKX₂TX₃X₄X₅X₆ (SEQ ID NO: 3) ist, und wobei X eine beliebige Aminosäure ist.

2. Zusammensetzung zur Verwendung nach Anspruch 1, wobei die Zusammensetzung einem Individuum verabreicht wird, das mit einem weiteren Therapeutikum behandelt wird.

3. Zusammensetzung zur Verwendung nach Anspruch 2, wobei das weitere Therapeutikum ein nukleotidischer-Reverse-Transkriptase-Inhibitor, ein nicht-nukleotidischer Reverse-Transkriptase-Inhibitor, ein Protease-Inhibitor, ein Entry-Inhibitor, ein Integrase-Inhibitor oder ein Budding-Inhibitor ist.

4. Zusammensetzung zur Verwendung nach Anspruch 1, wobei das Individuum mit einer Vielzahl an weiteren Therapeutika behandelt wird.

## Revendications

1. Composition comprenant un polypeptide pour une utilisation dans le traitement du VIH ou du SIDA, où le polypeptide est LKKTETQ (SEQ ID NO : 1), Ac-LKKTETQ-OH ou X₁LKX₂TX₃X₄X₅X₆ (SEQ ID NO : 3) et où X est un acide aminé quelconque.

2. Composition pour une utilisation selon la revendication 1, dans laquelle la composition est administrée à un sujet qui est traité avec un autre agent thérapeutique.

3. Composition pour une utilisation selon la revendication 2, dans laquelle l'autre agent thérapeutique est un inhibiteur nucléotidique de la transcriptase inverse, un inhibiteur non nucléotidique de la transcriptase inverse, un inhibiteur de la protéase, un inhibiteur de pénétration, un inhibiteur de l'intégrase, ou un inhibiteur de bourgeonnement.

4. Composition pour une utilisation selon la revendication 1, dans laquelle le sujet est traité avec une pluralité d'autres agents thérapeutiques.


### SEQUENCE LISTING

<110> ADISTEM LID
<120> METHOD OF TREATMENT OF HIV OR AIDS
<130> P126461EP00
<160> 17
<170> PatentIn version 3.5
<210> 1
   <211> 7
   <212> PRT
   <213> Homo sapiens
<400> 1
   Leu Lys Lys Thr Glu Thr Gln
   1               5
<210> 2
   <211> 44
   <212> PRT
   <213> Homo sapiens
<400> 2
   Met Ser Asp Lys Pro Asp Met Ala Glu Ile Glu Lys Phe Asp Lys Ser
   1               5                   10                  15
   Lys Leu Lys Lys Thr Glu Thr Gln Glu Lys Asn Pro Leu Pro Ser Lys
               20                  25                  30
   Glu Thr Ile Glu Gln Glu Lys Gln Ala Gly Glu Ser
           35                  40
<210> 3
   <211> 9
   <212> PRT
   <213> Artificial sequence
<220>
   <223> Consensus peptide sequence
<220>
   <221> misc_feature
   <222> (1)..(1)
   <223> Xaa can be any naturally occurring amino acid
<220>
   <221> misc_feature
   <222> (4)..(4)
   <223> Xaa can be any naturally occurring amino acid
<220>
   <221> misc_feature
   <222> (6)..(9)
   <223> Xaa can be any naturally occurring amino acid
<400> 3
   Xaa Leu Lys Xaa Thr Xaa Xaa Xaa Xaa
   1               5
<210> 4
   <211> 15
   <212> PRT
   <213> Homo sapiens
<400> 4
   Phe Asp Lys Ser Lys Leu Lys Lys Thr Glu Thr Gln Glu Lys Asn
   1               5                   10                  15
<210> 5
   <211> 15
   <212> PRT
   <213> Homo sapiens
<400> 5
   Phe Asp Lys Ala Lys Leu Lys Lys Thr Glu Thr Gln Glu Lys Asn
   1               5                   10                  15
<210> 6
   <211> 15
   <212> PRT
   <213> Homo sapiens
<400> 6
   Phe Asp Arg Ser Lys Leu Lys Lys Thr Glu Thr Asn Thr Glu Glu
   1               5                   10                  15
<210> 7
   <211> 15
   <212> PRT
   <213> Homo sapiens
<400> 7
   Phe Asp Lys Thr Lys Leu Lys Lys Thr Glu Thr Gln Glu Lys Asn
   1               5                   10                  15
<210> 8
   <211> 15
   <212> PRT
   <213> Homo sapiens
<400> 8
   Phe Asp Lys Ser Lys Leu Lys Lys Thr Asn Thr Glu Glu Lys Asn
   1               5                   10                  15
<210> 9
   <211> 15
   <212> PRT
   <213> Homo sapiens
<400> 9
   Phe Asp Arg Ser Lys Leu Lys Lys Thr Asn Thr Glu Glu Lys Asn
   1               5                   10                  15
<210> 10
   <211> 15
   <212> PRT
   <213> Homo sapiens
<400> 10
   Phe Asp Lys Thr Lys Leu Lys Lys Thr Glu Thr Ala Glu Lys Asn
   1               5                   10                  15
<210> 11
   <211> 15
   <212> PRT
   <213> Homo sapiens
<400> 11
   Phe Asn Arg Ala Lys Leu Lys Lys Thr Glu Thr Gln Glu Lys Asn
   1               5                   10                  15
<210> 12
   <211> 15
   <212> PRT
   <213> Homo sapiens
<400> 12
   Phe Asn Lys Ala Lys Leu Lys Lys Thr Glu Met Gln Glu Lys Asn
   1               5                   10                  15
<210> 13
   <211> 15
   <212> PRT
   <213> Homo sapiens
<400> 13
   Phe Asp Ala Lys Lys Leu Lys His Thr Glu Thr Asn Glu Lys Asn
   1 5 10 15
<210> 14
   <211> 15
   <212> PRT
   <213> Homo sapiens
<400> 14
   Phe Asn Gln Asn Asn Leu Lys His Thr Glu Thr Asn Glu Lys Asn
   1 5 10 15
<210> 15
   <211> 15
   <212> PRT
   <213> Homo sapiens
<400> 15
   Leu Asp Lys Ala Lys Leu Lys Ala Thr Glu Met Gln Glu Lys Asn
   1 5 10 15
<210> 16
   <211> 15
   <212> PRT
   <213> Homo sapiens
<400> 16
   Phe Asp Lys Ala Gly Leu Lys Lys Thr Glu Thr Glu Glu Lys Glu
   1 5 10 15
<210> 17
   <211> 6
   <212> PRT
   <213> Homo sapiens
<400> 17
   Leu Lys Lys Thr Glu Thr
   1 5